(19) Europäisches Patentamt
European Patent Office
Office européen des brevets

(11) Publication number: 0 077 651
A2

# (12) EUROPEAN PATENT APPLICATION

(21) Application number: 82305474.7

(22) Date of filing: 14.10.82

(51) Int. Cl.³: C 12 P 7/04
C 12 P 7/16, C 12 P 7/22
//C12R1/645

(30) Priority: 15.10.81 US 311507

(43) Date of publication of application:
27.04.83 Bulletin 83/17

(84) Designated Contracting States:
AT BE CH DE FR GB IT LI LU NL SE

(71) Applicant: CETUS CORPORATION
600 Bancroft Way
Berkeley, California(US)

(72) Inventor: Geigert, John
231 Southbrook Place
Clayton California 94517(US)

(72) Inventor: Neidleman, Saul Lewis
5377 Hilltop Crescent
Oakland California 94618(US)

(74) Representative: Bizley, Richard Edward et al,
BOULT, WADE & TENNANT 27 Furnival street
London EC4A 1PQ(GB)

## (54) Method of producing alpha, gamma-halohydrins and uses of the same.

(57) A method for producing alpha, gamma-halohydrins from cyclopropanes by enzymatic reaction avoiding the use of elemental halogens. A selected cyclopropane is reacted with a halogenating enzyme, an oxidising agent, and a halide ion source, generally at ambient conditions.

EP 0 077 651 A2

METHOD OF PRODUCING ALPHA, GAMMA-HALOHYDRINS AND USES OF THE SAME

This invention relates to a method for producing alpha, gamma-halohydrins from cyclopropanes and, more particularly, to an improved method for producing alpha, gamma-halohydrins from cyclopropanes by enzymatic reaction.

Alpha, gamma-halohydrins are commercially useful chemical intermediates in the manufacture of herbicides, detergents and polymers. For example, 3-bromo-1-propanol (I) is used in the manufacture of polymers employed as antimicrobial agents, flocculating agents, electroconductive agents in paper coatings or bile acid binding agents (Wagner et al, U.S. Patent No. 4,206,295). This compound is also used in the manufacture of substitued sulfonate herbicides (Arneklev, U.S. Patent No. 3,930,836). 3-Bromo-1-propanol (I) and 3-chloro-1-propanol (II) are used in the manufacture of flame retardant polyurethane polymeric compositions (Albright, U.S. Patent No. 4,054,544). The alpha, gamma-halohydrins are also important precursors for the synthesis of oxetanes (Bartock et al, Acta Chim. (Budapest) 70:133-142 (1971)).

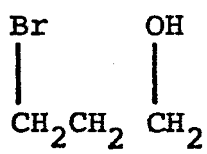
(I)

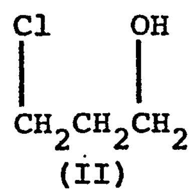
(II)

Alpha, gamma-halohydrins have been chemically prepared from cyclopropanes (LaLonde et al, J. Org. Chem. 37:2502-2505 (1972); Suryawanshi et al, Tetrahedr. Lettrs. 40:3595-3596 (1977)).

However, up to the present, typical methods of converting cyclopropanes to halogenated compounds have used free halogen, thus requiring expensive control procedures and equipment, and/or expensive reagents. Such processes also frequently produce undesirable side products, such as alpha, gamma-diols.

The enzymatic halogenating process of the present invention has several advantages over the present state of the art for producing alpha, gamma-halohydrins from cyclopropanes including the use of inexpensive and less dangerous inorganic halides are used rather than elemental halogen (for example, bromide ion is used rather than bromine), and the process also takes place generally at ambient temperatures and atmospheric pressure.

Very generally, the method of the invention produces alpha, gamma-halohydrins from cyclopropanes by providing in a reaction vessel a mixture of a halogenating enzyme, an oxidizing agent and a halide ion source. A cyclopropane is then introduced into the vessel and maintained in contact with the reaction mixture for a sufficient period of time to convert the cyclopropane to the desired alpha, gamma-halohydrin.

The present invention is based on the surprising discovery that the group of enzymes classified as haloperoxidases acts upon cyclopropanes to produce alpha, gamma-halohydrins. These products are characterised by the structural formula:

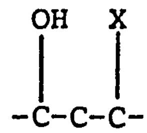

where X is selected from a group consisting of chloride, bromide or iodide. Although it was known in the prior art that haloperoxidases act upon active methylene or methine groups (Theiler et al, Science, 202:1096-1097 (1978)); Hager et al, Ann. N.Y. Acad. Sci. 244:80-93 (1975)), and that haloperoxidases act upon carbon-carbon double bonds (Neidleman et al, U.S. Patent No. 4,247,641), and that haloperoxidases act upon carbon-carbon triple bonds (Neidelman et al, U.S. Patent Application Serial No. 229,554, filed January 29, 1981), it has not heretofore been known that haloperoxidases act upon cyclopropanes.

The cyclopropanes useful in the process of the invention can be broadly defined as any hydrocarbon containing a three carbon ring represented by the following structural formula:

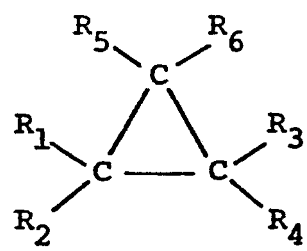

wherein each of $R_1$, $R_2$, $R_3$, $R_4$, $R_5$, and $R_6$ is selected from a group consisting of hydrogen and hydrocarbon. Representative cyclopropanes are:

| Cyclopropane | $R_1$ | $R_2$ | $R_3$ | $R_4$ | $R_5$ | $R_6$ |
|---|---|---|---|---|---|---|
| cyclopropane | H | H | H | H | H | H |
| methyl cyclopropane | $CH_3$ | H | H | H | H | H |
| phenyl cyclopropane | $C_6H_5$ | H | H | H | H | H |

The method of preparation comprises the introduction of a selected cyclopropane into a reaction mixture of a halogenating enzyme, a source of halide ion, and an oxidizing agent. The reaction proceeds rapidly under ambient comditions of temperature and pressure.

The halogenating enzyme employed in the invention is one of a number of haloperoxidase enzymes. Suitable haloperoxidase enzymes include chloroperoxidase derived from the microorganism Caldariomyces fumago, bromoperoxidase derived from algae, lactoperoxidase derived from milk, thyroid peroxidase derived from the thyroid, myeloperoxidase derived from leukocytes, and horseradish peroxidase derived from horseradish. Certain of these haloperoxidases are commercially available.

The preferred haloperoxidase depends upon the product or products desired. The halides that the given haloperoxidases can use are listed below:

| Haloperoxidase | Halides |
|---|---|
| myeloperoxidase | $Cl^-$, $Br^-$, $I^-$ |
| chloroperoxidase | $Cl^-$, $Br^-$, $I^-$ |
| lactoperoxidase | $Br^-$, $I^-$ |
| bromoperoxidase | $Br^-$, $I^-$ |
| thyroid peroxidase | $I^-$ |
| horseradish peroxidase | $I^-$ |

For ease of discussion, various aspects of the present invention are described with particularity, but not exclusively, in connection with the use of the preferred peroxidase, chloroperoxidase, derived from Caldariomyces fumago. The microorganism, Caldariomyces fumago, may be grown as a static or agitated, submerged culture in Czapek-Dox medium at room temperature for 3 to 10 days by conventional methods. The halogenating enzyme, chloroperoxidase, is prepared from an aqueous homogenate of the mycelial pads of the microorganism grown under static conditions or from the filtrate of the microorganism grown under static or agitated submerged culture conditions.

The halogenating enzyme may also be used in an immobilized form. Processes for enzyme

immobilization are familiar to those skilled in the art, and include reacting either a solution of the enzyme or a suspension of enzyme containing cells with one of a broad range of organic and inorganic supports. Included among these are polyacrylamide, ethylene-maleic acid copolymers, methacrylic-based polymers, polypeptides, styrene-based polymers, agarose, cellulose, dextran, porous glass beads, and aluminum or titanium hydroxide. Enzymes in this form have increased stability, extended life and usefulness, and recoverability. Reactions employing immobilized enzymes may be run in columns or reaction tanks.

In addition to the halogenating enzyme, a source of inorganic halide and an oxidizing agent are required in the reaction mixture. A preferred oxidizing agent is hydrogen peroxide, which may be added directly to the mixture in a single batch addition, or in a continuous slow feed. It may alternatively be generated as a slow feed <u>in situ</u> by the use of a hydrogen peroxide-producing enzyme system. Such enzyme systems are well known in the art, and include glucose-1-oxidase in the presence of D-glucose, pyranose-2-oxidase or glucose-2-oxidase in the presence of D-glucose, D- and L-amino acid oxidases in the presence of D- and L-methionine, methanol oxidase in the presence of methanol, and diamine oxidases in the presence of histamine. The hydrogen peroxide-generating system may be present in the non-immobilized or immobilized state as with the halogenating enzyme. The hydrogen peroxide may also be generated by a chemical reaction, such as the anthraquinone or isopropyl alcohol oxidation processes.

The hydrogen peroxide is present preferably in a molar ratio of from about 0.5:1, most preferably in a ratio of about 1:1 or less with respect to the

cyclopropane. The molar ratio preferences refer to the average presence of hydrogen peroxide during the reaction. The actual molar ratio will usually vary during the reaction and the molar ratio at any particular time may be above or below the ranges cited. Other suitable oxidizing agents include organic peroxides, such as methyl, ethyl, or butyl peroxides.

The halogen source may be any of the watersoluble halide salts. The preferred halogen sources are the chloride, bromide, and iodide salts of the alkali metals, sodium and potassium. The salts are present in the reaction mixture at a level sufficient to provide a slight excess of halide ion with respect to the stoichiometric amount required for the reaction.

The reaction is conducted within the pH range of from about 2.2 to about 8.0. The pH of the reaction may be maintained within the desired range by use of a buffering agent. Suitable buffers include sodium or potassium phosphate based systems. Other suitable techniques besides buffering may be used for pH control and adjustment. The reaction is preferably conducted in an aqueous medium. While some of the cyclopropanes that can be converted by the process are substantially insoluble in an aqueous medium, the reaction, nevertheless, occurs satisfactorily under conditions of mixing, or other modes of dispersion which provide sufficient substrate solubility for the reaction.

It is also contemplated that the reaction can be conducted in the presence of low levels of organic solvents to increase substrate solubility. The reaction is preferably conducted under aerobic conditions and in the temperature range of 15°C to about 50°C, preferably about 20°C to about 30°C.

As previously indicated, the components of the reaction mixture, namely the cyclopropane, the

halogenating enzyme, the oxidizing agent, the halide ion source, and the buffering agent, may be simply mixed together in water or mixed aqueous or organic media, and agitated for a period of from about 30 seconds to about 1 hour to obtain the halogenated products. Cyclopropanes, such as cyclopropane and methyl cyclopropane, which are gaseous, can be reacted upon by simply passing the gaseous cyclopropanes through the reaction mixture.

Analysis of the halogenated products is accomplished by gas chromatography (GC) using mass spectrometric (MS) detection. A 6-foot glass column packed with Tenax-GC, 80/100 mesh (Applied Sciences Lab., State College, Pennsylvania), operated from 100°C to 250°C at 10°C/minute temperature programming, is attached to a Finnigan 4021 GCMS (Finnigan Corp. Sunnyvale, CA). Detection, quantitation and structure identification are made possible by operating the mass spectrometer at 70 eV electron impact ionization. Reaction mixtures are analyzed by direct injection (10 µℓ) of the aqueous reaction mixtures, and by ethyl ether extraction, concentration and then injection.

The following Examples are intended only further to illustrate the invention and are not intended to limit the scope thereof.

Example 1

Potassium bromide (50 mg) and potassium phosphate buffer (8 ml, 0.1 M, pH 3.0) were mixed together in a 100 ml Pyrex flask at room temperature and room pressure. Gaseous methyl cyclopropane (purchased from I.C.N./K and K, Irvine, CA) was continuously bubbled into the mixture. The haloperoxidase enzyme, chloroperoxidase (0.1 ml), was added, followed by addition of dilute hydrogen peroxide (0.4 ml of 3%

solution). The reaction was concluded 30 minutes after the addition of the last reagent.

The chloroperoxidase was prepared as follows:

Mycelial pads of Caldariomyces fumago (ATCC 16373) were grown on potato agar slants. Sliced potato (200 g) was cooked in distilled water (500 ml) for 40 minutes and then strained. A solution of glucose (21 g) and agar (20 g) in distilled water (500 ml) was added to the strained solution. The pH was adjusted to 6.8 and the volume was brought to 1 liter with distilled water. The medium was sterilized at 121°C for 15 minutes.

The organism was inoculated on the potato agar slants, produced in accordance with the above procedure, and was grown for about one week at room temperature. The organism was then used to inoculate a soybean-glucose medium (50 ml). The soybean-glucose medium was prepared by adding, to 1 liter of distilled water, extraction process soybean meal (30 g), glucose (30 g), and $CaCO_3$ (7 g). The medium was sterilized at 121°C for 30 minutes and was then inoculated with the organism after cooling.

The organism was grown for 4-5 days on a rotary shaker at 25°C. 5 ml of this material was used to inoculate a 500 ml Erlenmeyer flask containing 100 ml of a modified Czapek-Dox medium prepared by adding the following to 1 liter of distilled water: $NaNO_3$ (3 g), $KH_2PO_4$ (1 g), KCl (0.5 g), $MgSO_4 \cdot 7H_2O$ (0.5 g), $FeSO_4 \cdot 7H_2O$ (10 mg), and glucose (40 g). The medium was sterilized at 121°C for 20 minutes prior to inoculation with the organism.

The organism was grown under static conditions at room temperature for 5-7 days. The black mycelial pads which formed were collected, rinsed with distilled water, and stored in plastic bags in a freezer at -10°C for subsequent use.

The halogenating enzyme was prepared by grinding 6 mycelial pads (prepared in accordance with the above procedures) with 60 g acid-washed sand and 60 ml distilled water for 2 minutes in a Virtis 45 homogenizer. The homogenate was centrifuged while cold and the supernatant solution was used as the source of the halogenating enzyme, chloroperoxidase.

The final chloroperoxidase supernatant was filtered through Whatman No. 1 paper at room temperature. The filtrate was concentrated about 10-fold using a rotary film evaporator at reduced pressure and temperature (<35°). The concentrate was chilled at 0°C in an ice bath, and pre-chilled (0°) ethanol was added until 45% ethanol (v/v) was reached. The mixture was stirred vigorously for 15 minutes, and then centrifuged at -10°C (at 15,000 g) with a 55-34 rotor in a Sorval RC-5 Superspeed for 15 minutes. The black sediment was discarded. To the centrifugate cooled at 0°C, was added additional prechilled ethanol to give 65% ethanol (v/v). The mixture was slowly stirred for 30 minutes at 0°C, and then centrifuged as before. The centrifugate was discarded, and the precipitate containing the chloroperoxidase activity was dissolved in 1 ml of 0.05 M potassium phosphate buffer (pH 7). The enzyme solution was stored at -20°C.

The products were detected and identified by gas chromatography-mass spectrometry (GCMS). The reaction of mixtures were extracted with ethyl ether (3 x 5 ml volumes). The ethyl ether layer was separated and then taken down to a 1 ml volume by blowing nitrogen over it at 40°C, 10 µℓ of this concentrated extract being injected into a Finnigan 4021 GCMS, equipped with a 6 foot by 1/4 inch coiled, glass column, packed with Tenax-GC (80/100 mesh). Flow through the column was set at 30 ml per minute;

the column temperature was programmed from 100°C to 250°C at a rate of 10°C/minute; the mass spectrometer was set at 70eV electron impact ionization.

Two products were detected.

The major product had a GC retention time of 11.0 minutes and showed the mass spectrum diagnostic for 1-bromo-3-butanol: molecular mass ion at mass 152 and 154 (1:1 in intensity), indicating 1 bromine atom on the molecule; major fragment mass ions at mass 137 and 139 (1:1 in intensity; loss of $CH_3$ from the molecular ion), at mass 93 and 95 (1:1 in intensity; the $CH_2Br^+$ ion), at mass 73 (loss of Br from the molecular ion), at mass 72 (loss of HBr from the molecular ion), and at mass 45 (the $CH_3CH = OH^+$ ion).

The minor product had a GC retention time of 11.5 minutes and showed the mass spectrum diagnostic for 3-bromo-1-butanol: molecular mass ion at 152 and 154 (1:1 in intensity), indicating 1 bromine atom on the molecule; major fragment mass ions at mass 107 and 109 (1:1 in intensity; the $CH_3$ $CH = Br^+$ ion), at mass 122 and 124 (loss of $CH_2O$ from the molecular ion), and at mass 80 and 82 (1:1 in intensity; the $HBr^+$ ion).

A total yield of 15 mg of alpha, gamma-bromohydrin product was obtained.

## Example 2

The procedure of Example 1 was followed, except that lactoperoxidase (purchased from Sigma Chemical Company, St. Louis, MO; Catalog No. L-7129) was substituted for chloroperoxidase, and the pH of the buffer was 6.0 instead of 3.0.

A total yield of 6 mg of alpha, gamma-bromohydrin product was obtained, as described in Example 1.

## Example 3

The procedure of Example 1 was followed,

except that potassium chloride was substituted for potassium bromide.

Two products were obtained.

The major product had a GC retention time of 9.1 minutes and showed the mass spectrum diagnostic for 1-chloro-3-butanol: molecular mass ion minus one hydrogen at mass 107 and 109 (3:1 in intensity), indicating 1 chlorine atom on the molecule; major fragment mass ions at mass 93 and 95 (3:1 in intensity; loss of $CH_3$ from the molecular ion), at mass 72 (loss of HCl from the molecular ion), and at mass 45 (the $CH_3CH = OH^+$ ion).

The minor product had a GC retention time of 9.4 minutes and showed the mass spectrum diagnostic for 3-chloro-1-butanol: molecular mass ion minus one hydrogen at mass 107 and 109 (3:1 in intensity), indicating 1 chlorine atom on the molecule; a major fragment mass ion at mass 63 and 65 (3:1 in intensity; the $CH_3CH = Cl^+$ ion).

A total yield of 8 mg of alpha, gamma-chlorohydrin product was obtained.

Example 4

The procedure of Example 1 was followed, except that phenyl cyclopropane was substituted for methyl cyclopropane. Since phenyl cyclopropane is a liquid cyclopropane, it was added to the flask (0.05 ml) at the start rather than bubbled in.

One product was obtained. This product had a GC retention time of 15 minutes and showed the mass spectrum diagnostic for 3-bromo-1-phenyl-1-propanol: molecular mass ion at mass 214 and 216 (1:1 in intensity), indicating 1 bromine atom on the molecule; major fragment mass ions at mass 135 (loss of Br from the molecular ion) and at mass 107 (the $\phi CH = OH^+$ ion).

A total yield of 13 mg of alpha, gamma-bromohydrin product was obtained.

Example 5

The procedure of Example 4 was followed, except that horseradish peroxidase (purchased from Sigma Chemical Company; 10 mg; Catalog No. H-5130) was substituted for chloroperoxidase, and the pH of the buffer was 6.0 instead of 3.0.

One product was obtained. This product had a GC retention time of 16.6 minutes and showed the mass spectrum diagnostic for 3-iodo-1-phenyl-1-propanol: molecular mass ion at mass 260 not observed; major fragment mass ions at mass 135 (loss of I from the molecular ion) and at mass 107 (the $\phi CH = OH^+$ ion).

It may be seen, therefore, that the invention provides an improved process for the manufacture of alpha, gamma-halohydrins from cyclopropanes. The reaction proceeds enzymatically and does not require the use of free halogen.

The invention includes an alpha, gamma-halohydrin which has been produced by the process of the invention and for use in the manufacture of herbicides, detergents or polymers, for example for use in the manufacture of polymers employed as antimicrobial agents, flocculating agents, electroconductive agents in paper coatings or bile acid binding agents, for use in the manufacture of substituted sulfonate herbicides, for use in the manufacture of flame retardant polyurethane polymeric compositions, for use in the synthesis of oxetanes.

Various modifications of the invention in addition to those described herein will be apparent to those skilled in the art and are included within the invention.

## REFERENCES

U. S. Patent No. 4,206,295 - Wagner, et al.
U. S. Patent No. 3,930,836 - Arneklev
U. S. Patent No. 4,054,544 - Albright
U. S. Patent No. 4,247,641 - Neidleman, et al.

Bartok, M., K. Lang-Lakos and J. Bozoki-Bartok, Acta Chimica Academiae Scientiarum Hungaricae, Vol. 70, 1971, pp. 133-142.

LaLonde, R. T. and P. F. Ferrara, Journal of Organic Chemistry, Vol. 37, 1972, pp. 2502-2505.

Suryawanshi, S. N. and U. R. Nayak, Tetrahedron Letters, Vol. 40, 1977, pp. 3595-3596.

Theiler, R., J. C. Cook and L. P. Hager, Science, Vol. 202, 1978, pp. 1094-1096.

Hager, L. P., P. F., Hollenberg, T. Rand-Meir, R. Chiang and D. Doubek, Annals of the New York Academy of Science Vol. 244, 1975, pp. 80-93.

CLAIMS:

1. A method for producing an alpha, gamma-halohydrin from a cyclopropane, comprising reacting together a halogenating enzyme, an oxidising agent, a halide ion source and a selected cyclopropane for a sufficient period of time to convert said cyclopropane to a alpha, gamma-halohydrin.

2. A method in accordance with claim 1, wherein the halogenating enzyme is a peroxidase derived from the microorganism Caldariomyces fumago, algae, milk, thyroid, leukocytes or horseradish.

3. A method in accordance with claim 1 or claim 2, wherein the oxidising agent is hydrogen peroxide.

4. A method in accordance with claim 13, wherein the hydrogen peroxide is present at a molar ratio to the cyclopropane of from about 0.5:1 to about 50:1.

5. A method in accordance with claim 3 or claim 4, wherein the hydrogen peroxide is generated *in situ*.

6. A method in accordance with any one of claims 1 to 5, wherein the halide ion source is a water soluble halide salt.

7. method in accordance with any one of claims 1 to 6, wherein the reaction is conducted at a pH range of from about 2.2 to abour 8.0.

8. A method in accordance with claim 1, wherein the halogenating enzyme is derived from the microorganism Caldariomyces fumago or seaweed or is lactoperoxidase, the oxidizing agent is hydrogen peroxide, the halid ion source is a chloride, bromine or iodide salt of sodium or potassium, and the reaction takes place in an aqueous environment at ambient conditions of temperature and pressure.

9. A method in accordance with any one of claims 1 to 8, wherein the cyclopropane is cyclopropane, methyl cyclopropane or phenyl cyclopropane.

10. An alpha, gamma-halohydrin which has been

produced by a method as claimed in any one of claims 1 to 9 and for use in the manufacture of herbicides, detergents or polymers, for example, for use in the manufacture of polymers employed as antimicrobial agents, flocculating agents, electroconductive agents in paper coatings or bile acid binding agents, for use in the manufacture of substituted sulfonate herbicides, for use in the manufacture of flame retardant polyurethane polymeric compositions, or for use in the synthesis of oxetanes.